# EUROPEAN PATENT APPLICATION

(11) **EP 3 674 951 A1**
(43) Date of publication of application: **01.07.2020**
(21) Application number: 18215982.2
(22) Date of filing: 31.12.2018
(51) Int. Cl.: G06F 21/62, G06F 21/32, A61B 5/16, H04L 29/06, H04L 9/32

(54) **SYSTEM AND METHOD OF OBTAINING AUTHENTICATION INFORMATION FOR USER INPUT INFORMATION**

(71) Applicant: Nederlandse Organisatie voor toegepast- natuurwetenschappelijk onderzoek TNO, 2595 DA 's-Gravenhage (NL)
(72) Inventor: VAN REST, Jeroen Hendricus Conelis, 2595 DA 's-Gravenhage (NL)
(74) Representative: V.O.

(57) **Abstract**

A terminal is used to obtain authentication information associated with user input information. The terminal measures a set of values of biometric properties of behavior of a person at the terminal, using one or more biometric behavior sensors of the terminal. An indication of a time interval is provided from which the set of values has to be used for obtaining the authentication information for the user input information. Parameters of a biometric of behavior template are read from a user domain storage device, such as a smartphone, to which access is enabled only under control of the person. The parameters define a predetermined class of sets of values of biometric properties that have been determined to occur for the person when the person is in a predetermined type of mental state, or a computation for computing a score value from the set of values that the set of measured values belongs to said class. The parameters are used in the terminal to determine whether or not the set of values in said time interval are within the predetermined class, and/or to compute a score value that the set of values in the time interval belongs to said class, or the terminal may cause a computing device associated with the user domain storage device to do so.

## Description

### Field of the invention

The invention relates to a system and method of using a terminal to obtain authentication information associated with user input information, such as information that a person that provided the information lied when the person provided the information.

### Background

A lie detector measures biometric aspects of behavior of a human person to determine whether the measured biometric aspects correspond to a predetermined class of measured values. In psychological terms, the lie detector distinguishes whether the measured biometric aspects correspond to an mental state wherein the human person believes that a statement that he or she makes is true or an mental state wherein the human person believes that the statement is not true. In technical terms, this corresponds to classification of sets of measured values of biometric behavior properties. In most applications of lie detection, the statement is an answer given by the person to a question from a human interrogator that is known to want a truthful answer. The detection is performed using sensors for sensing biometric behavior properties of the person that answers the question, at the time at which he or she answers the question, and/or in the time period from asking the question to answering it.

The measurements of biometric behavior properties may include measurements of changes in physiological body properties, such as heart rate, electrical skin resistance, breathing frequency and measurements of properties of sound produced by the person such as pitch variation, and measurements of properties of body movements such as eye blinking frequency. In general, biometric behavior properties can be distinguished from biometric identification properties. The latter are independent of the person's mental state.

Machine based lie detection involves classification of the measurements of such biometric behavior properties. Machine based lie detection determines whether the measurements fall within a predetermined class, or computation of a likelihood value that the measurements fall into such a class. As noted, the resulting classification or likelihood values do not represent whether the statement is (likely) true or not, but merely indicate whether the biometric behavior properties belong to a predetermined class of measurement results, or the likelihood that they belong to such a predetermined class.

It has been found to be preferable to use different class definitions for different human persons for lie detection. To realize this, person specific parameters to control the computation of likelihoods and/or classification may be used. For example, parameters may specify threshold values, weights for different measurements, argument offsets and scale factor of functions to be applied to different measurements etc. to be used in the likelihood computation and/or classification from the measurements of biometric behavior properties. Such parameters have to be determined prior to classification using a training procedure. The resulting parameters are subsequently used in a classification procedure, wherein the lie detector uses such parameters for the classification or computation of likelihoods.

To determine the parameters, the person makes test statements, including statements that the person is known to believe that they are true and statements that the person is known to believe that they are false, and test measurements of the person's biometric behavior properties are measured in time intervals wherein the person makes these test statements. These test measurements are used to determine parameters values for the classification or computation of likelihoods. Generally, the parameters are selected so that, when used to compute the likelihood and/or classification from the test measurements, the results are consistent with the known belief of truth and falsehood of the statements.

In the prior art such lie detection procedures are used in specific situations, for example in the course of a legal investigation to support or question the reliability of a witness statement during interrogation. The procedure takes considerable time and expertise, which limits its applicability.

### Summary

Among others it is an object to facilitate the use of a terminal to obtain authentication information associated with user input information provided at the terminal.

According to one aspect, a method of using a terminal to obtain authentication information associated with user input information is provided, the method comprising
- measuring a set of values of biometric properties of behavior of a person at the terminal;
- receiving an indication of a time interval from which the set of values has to be used for certifying the user input information;
- reading parameters of a biometric of behavior template from a user domain storage device, to which access is enabled only under control of the person,
- wherein the parameters define a predetermined class of sets of values of biometric properties that have been determined to occur for the person when the person is in a predetermined type of mental state, or a computation for computing a score value from the set of values that the set of measured values belongs to said class;
- using the parameters in the terminal, a computing device
associated with the user domain storage device to determine whether or not the set of values in said time interval are within the predetermined class, or computing a score value that the set of values in the time interval belongs to said class.

In this way, it is possible to use a terminal to obtain a classification of the person's mental state when the user provides input information, e.g. whether or not state corresponds to values of biometric properties of behavior when the user is lying. As used herein a classification is a determination that the set of values is in a predetermined class or a score for this. The user domain storage device is a portable physical information carrier supplied by the person, or a remote storage device to which access has been given under control of the person. By using a previously prepared portable biometric behavior template from the user domain storage device at the terminal, the time needed to set up usual detectors is avoided. Preferably, the terminal uses a plurality of different biometric behavior sensors, and the biometric behavior template provides for use of a combination of such a plurality. Moreover, by making the use of the biometric behavior template dependent on enabling of access to the user domain storage device, the risk of abuse beyond control of the user is reduced. Enabling access may involve presentation of the portable physical information carrier by the person, or supplying access control information from the person.

In addition to the biometric behavior template for a person, the user domain storage device may comprise a biometric identification template for that person, and the terminal may be configured to use the biometric identification template to measure biometric identification properties of the person and verify the identity of the person as a condition for performing the classification. It should be noted that, biometric behavior properties and biometric identification properties are distinct, even if they can be measured with the same sensors. Generally, biometric identification properties are static properties that do not change, whereas biometric behavior properties must be able to vary from time to time for the same person. Thus for example biometric identification properties may involve a fingerprint image or a retina scan, whereas biometric behavior properties may involve a video capturing facial expressions.

In an embodiment, it is made possible to update the parameters of a biometric of behavior template when it is discovered later that the classification result was erroneous, e.g. that the person that supplied the input information lied, whereas the classification result did not indicate a lie. To do so, the set of values of the biometric properties of behavior that was measured when the input was supplied is stored for later use, associated with an identifier of that input. When a person discovers the error, that person may input a corrected classification in relation to the identifier. Subsequently, when the portable physical information carrier is again presented, or access control information is supplied, the parameters of a biometric of behavior template may be updated, by modification based only on the set of measured values associated with the identifier and its corrected classification, or by using this set and other sets with their recorded classifications to select the update. The update may even involve training the template anew. This embodiment provides for a method of using a system of terminals to certify user input information, wherein a first terminal of the system performs the method according to claim 1 or 2, the first terminal of the system further recording the set of values in association with an identifier of an event wherein the user input information was input in the user domain storage device, the method further comprising
- storing a subsequent feedback in association with the identifier in a server of the system, the subsequent feedback indicating that the person lied at the event;
- executing the method of claim 1 by a second terminal of the system, wherein, when the person gives the second terminal access to the user domain storage device, and before the second terminal uses the parameters are used in said step of using the parameters, the second terminal updates the parameters based on the recorded set of values in and the subsequent feedback associated with the identifier that was stored in the server, or causes the portable physical information carrier or the computing device associated with the storage device to do so.

The time interval from which the set of values is obtained preferably contains the time point or time interval for which it is determined that the person provides the user input information and a time interval preceding that time point, in which the person prepares the user input information, e.g. a time interval between a time point at which a question is posed to which the user input information is an answer and the time point at which the person provides the user input information in response. However, the time interval may also comprise a post response time interval.

The parameters of the biometric of behavior template may take different forms, dependent on the classification technology that is used. For example, the parameters may include one or more sets of measurements of values of biometric properties of behavior, each combined with an indication of the class associated with that set, or parameters of functions used to compute a classification score, such as parameters of support vector machine score functions, or of a neural network.

The portable physical information carrier may be a smart phone, or a smart card for example. The portable physical information carrier may supply all the parameters of the biometric of behavior template, but alternatively it may supply part of the part of the parameters, another part being supplied by from a central storage device, e.g. in a server. As another alternative, all the parameters of the biometric of behavior template may be supplied from such a central storage device when the person provides information that is used to control approves access to that storage device. Part of the biometric of behavior template may be accessible without requiring controlled access, but it is preferred to prevent that both sets of biometric behavior property measurements and the classifications associated with these sets are accessible without requiring controlled access. For example, sets of biometric behavior property measurements might be stored in the central storage device and the information about the classification of these sets might be stored in the portable physical information carrier or vice versa.

### Brief description of the drawing

These and other advantageous aspects will become apparent from a description of exemplary embodiment with reference to the following figures.
Figure 1 shows an overview of a system with devices that use lie detectors
Figure 2 shows a flow chart of an aspect of operation of the system
Figure 3 shows a flow chart of a further aspect of operation of the system
Figure 4 shows a flow-chart of an embodiment of operation of a terminal

Detailed description of exemplary embodiments.

Lie detection has both awkward and useful aspects for a person that submits to lie detection. Obviously, lie detection is an infringement of mental privacy. On the other hand if a person can achieve a desirable goal by convincing others that a statement is not a lie, the person may voluntarily submit to lie detection. Submission to lie detection to prove one's innocence from a punishable act is a familiar example, but lie detection can also be convenient in more day to day situations such as being allowed to pass luggage through customs quickly based on a statement that the luggage contains nothing to declare, or to obtaining access to a secured space without a body search based on a statement that a person carries no banned objects, or has not engaged in activities that would prohibit access.

However, present day lie detection is too time consuming for such day to day use. It would be desirable to make the use of lie detector less time consuming. Such a development may make lie detection more pervasive, but it may also raise concerns of abuse to obtain private information when the interrogated person is unaware that lie detection is used, or used without permission for the purposes of the interrogator. Therefore it is desirable to make use of lie detector less time consuming, in a way that makes unauthorized use difficult, if not impossible.

Lie detection per se, in the abstract sense of determining whether a person is in mental state wherein the person believes that a statement made by the person is not true, is not a technical process. But a technical implementation of lie detection involves specific types of sensor measurements, more specifically measurements of biometric behavior properties. In contrast, measurements of static biometric properties, such as nose length measurements, which are not behavior properties, are in practice not sufficient. Moreover, the technical implementation involves a computation of a similarity between sets of measurements of the biometric behavior properties provided as training examples and the measurements of the biometric behavior properties to determine class, or compute a similarity score. Such a computation of a similarity may directly involve comparing with the training examples or indirect comparison, by computing functions such as support vector machine scores or neural network signals, that have been determined by means of the training examples. Parameters for such direct or indirect comparisons will be referred to as a biometric behavior template. The technical implementation should provide for user control over the use of the biometric behavior template.

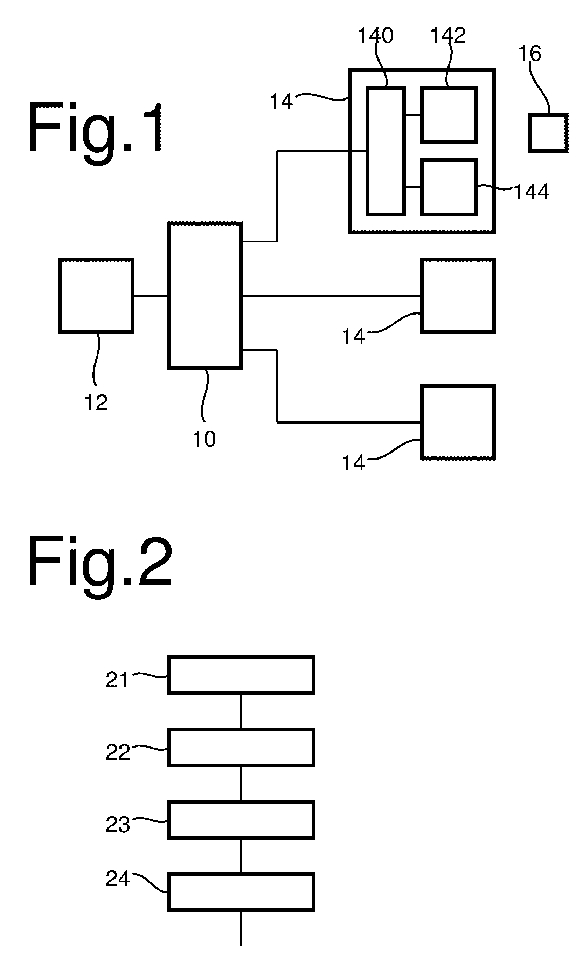

Figure 1 shows an overview of a system with devices that use lie detection. The system comprises a central server 10, an enrollment device 12, a plurality of terminals 14 and a portable physical information carrier 16. Portable physical information carrier 16 is an example of a user domain storage device, that is, a device to which access is enabled only under control of a specific person. The user domain storage device may be part of a user domain processing device, that comprises a user domain processor and the user domain storage device. For example, portable physical information carrier 16 comprises a memory and a processor. Portable physical information carrier 16 may be a smart phone or a chip card like a smart card for example. Preferably, information carrier 16 is configured so that external access to its memory is not possible without intervention of the processor. Enrollment device 12 is configured to cause information to be written on physical information carrier 16, either by supplying the information to information carrier 16, or by causing the processor of physical information carrier 16 to generate the information and store it. Instead of portable physical information carrier 16 another type of user domain storage device may be used, such as a storage device with encrypted information to which only the person has a decryption key, or a storage device that requires a password or biometric identification data to enable access, but a description using portable physical information carrier 16 will be disclosed by way of example.

Terminals 14 are configured to obtain information from physical information carrier 16. Terminals 14 are coupled to central server 10, which may be a computer or a distributed computer system. Central server 10 comprises a storage device (not shown). Terminals 14 may be coupled to central server 10 e.g. via the Internet. Each terminal 14, may have the same structure as the other terminals 14. By way of example, one of terminals 14 is shown in more detail, comprising a computer 140, an optional statement input device 142 and a biometric behavior sensing device 144. When a portable physical information carrier 16 is used, computer 140 may comprise a communication unit(not separately shown) for communicating with portable physical information carrier 16, using e.g. electrical contacts, near field communication or a Bluetooth interface or similar interface. Enrollment device 12 may have a similar structure as terminals 14. In an embodiment enrollment device 12 and terminals 14 may be used interchangeably as enrollment device and terminal. Although a single enrollment device 12 is shown by way of example, a plurality of enrollment devices may be used instead.

Dependent on the implementation, biometric behavior sensing device 144 may comprise one or more different sensors, e.g. a camera, a sound signal detector, a contact based sensor, a keyboard etc. Computer 140 may be configured (e.g. programmed) to derive various measurements using input from the sensors. As used herein, a sensor may refer to a combination of a sensing device and a computer program to derive measured values from an output signals of a sensing device, or to the a sensing device per se. For example, computer 140 may be configured to use input from the image sensor to detect movements and events such as head movement, motion of other user limbs, eye blinking events, eye ball movement, mouth deformation, other time dependent face deformation, face color variation etc. in video input with images that contain the face of the user. Computer 140 may be configured to use input from the sound signal detector to detect pitch and loudness in the sound signal, variation of the pitch and/or loudness, words determined by applying voice recognition to the sound signal, the presence of meaningless interjections in the sound signal such as stop words or pause events for breathing. Contact based sensors may be used to detect time dependent heartbeat, typing frequency on a keyboard, electrical skin resistance of a finger on a key or on a dedicated sensor, time dependent pressure variation e.g. during typing etc. Furthermore, biometric behavior sensing device 144 may comprise a fMRI scanner or other type of scanner. The biometric fMRI scanner or other type of scanner may be configured to perform scans of positions in the brains and to determine one or more values of measurements of predetermined types of brain activity at one or more predetermined regions in the brain.

Optional statement input device 142 is configured to input information supplied by the user. Computer 140 may be configured to process this information. The information or processed information will be referred to as the "statement". Statement input device 142 may share an input device with biometric behavior sensing device 144. E.g. statement input device 142 may comprise a keyboard for typing the statement or a sound signal input device for recording the statement.

The information derived for statement input differs from that derived for biometric behavior sensing. The information derived for statement input typically uses only one input device, and only meaning carrying aspects of the input from the input device, such as key codes of typed keys, speech recognition data or compressed speech data. In contrast, information derived for biometric behavior sensing typically uses a plurality of aspects of the input that are independent of the meaning carrying aspects used by statement input device 142 and/or more than one different input device.

Figure 2 shows a flow chart with an overview of operation of the system. When a human user presents her or himself at enrollment device 12, enrollment device 12 generates a biometrics behavioral template for use to classify biometric behavior sensing data. The generation of the template will usually be executed under supervision of a human supervisor, but in some embodiments it may be performed automatically.

The template generation involves a first step 21 wherein enrollment device 12 measures time dependent biometric behavior properties of the user under various different conditions, for example when the user answers different questions posed by the supervisor. In one example the supervisor may first inform the user of a complex of facts (e.g. a series of events) that should be assumed to be true and subsequently ask questions about those facts from that complex of facts.

The supervisor may ask the user to find and tell lies in response to part of the questions, or to hide certain facts from the complex of facts in answers to a series of questions. Enrollment device 12 may receive input from the supervisor for each answer indicating whether the user lied or not, for example based on whether the user was asked to find and tell a lie to the question or not, or whether the answer is at variance with the complex of facts. Furthermore, enrollment device 12 may receive input from the supervisor that indicates time points or time intervals during which the user supplied the answers and optionally time points or time intervals during which questions were asked to which these answers were a response. Alternatively, enrollment device 12 may be configured to perform part or all of the tasks of the supervisor automatically.

For each answer, enrollment device 12 may form a set of values of measured biometric behavior properties of the user in a time interval in which the answer is given and or prepared. The set of values may be based on measurements at a single time point in the time interval, or on the evolution of the measurements of the measured biometric behavior properties during the time interval.

In a second step 22 enrollment device 12 and/or physical information carrier 16 determine parameters for computation classifications or likelihoods of classifications based on the sets if values of measurements of the dependent biometric behavior properties and the input when the user answered questions and whether the user lied or not obtained in first step 21.

As part of first or second step 21, 22 enrollment device 12 converts the sets of values of biometric behavior properties obtained at the time points or time intervals of the answers into machine independent sets of values of measurement data. For biometric behavior properties like eye blinking rate, no conversion may be needed to obtain machine independent measurement data, but for other properties enrollment device 12 may need to use calibration data to obtain device independent data. Next, enrollment device 12 and/or physical information carrier 16 determines parameters for computation of classifications or likelihoods or similar score of classifications that will classify the machine independent set of value measurement data, according to the input whether the user lied or not, or that will optimize likelihood scores according to this input. In an embodiment, the classification parameters may be determined using the different answers as independent training examples.

The method of determining the parameters depends on the classification method that will be used. For example, if support vector machine classification will be used, the machine independent measurement data may be binned according to a predetermined feature dictionary and support vectors may be determined. If a neural network will be used, a known neural network training method may be used. In other embodiments clustering may be used.

Furthermore, enrollment device 12 may perform a user identification process, to obtain an identification of the user, which is associated with user identification information. Alternatively, enrollment device 12 or physical information carrier 16 may derive user identification information directly from measurements performed on the user.

In a third step 23, enrollment device 12 and/or physical information carrier 16 generates a machine independent biometrics behavioral template containing the parameters. In the embodiment wherein user identification information is derived, enrollment device 12 and/or physical information carrier 16 executes a fourth step 24 of storing the biometrics behavioral template in association with user identification information on physical information carrier 16.

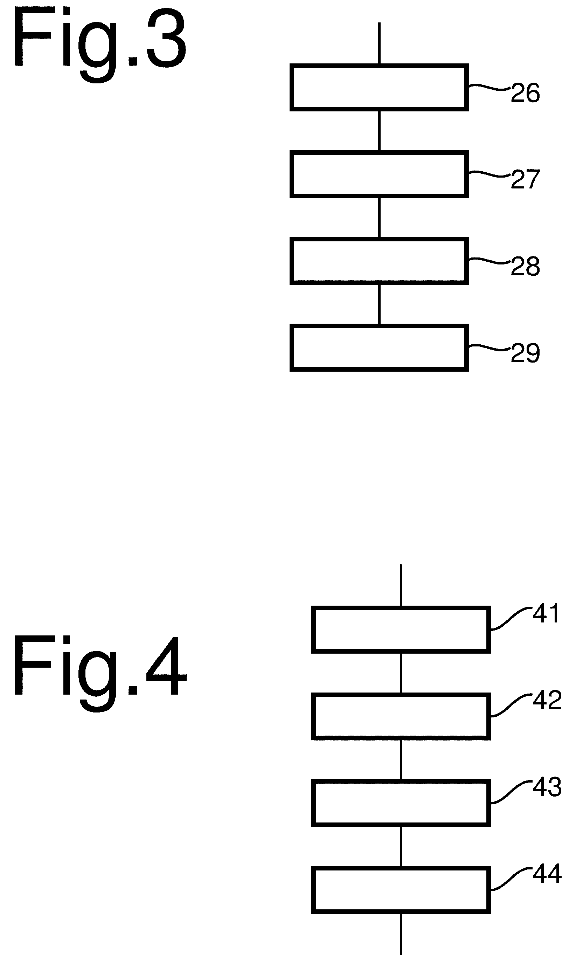

Figure 3 shows a further process executed by any terminal 14 and physical information carrier 16 later, when the human user presents physical information carrier 16 at that terminal 14. The terminal 14 executes a sixth step 26, of measuring time dependent biometric behavior properties of the user during one or more time intervals, using biometric behavior sensing device 144. As in the case of the steps performed by enrollment device 12, the input may involve human supervisor, which asks one or more questions from the user and provides input to terminal 14 indicating time point(s) or time interval(s) wherein the user answers the questions. Like enrollment device 12 in first or second step 21, 22, terminal 14 converts the biometric behavior properties into a set of values of biometric behavior properties, each obtained at the time point or time interval of a respective answers. Each set of values may be converted into machine a set of values of independent measurement data of biometric behavior. As used herein this machine independent biometric behavior measurement data will be said to be associated with the answer. Physical information carrier 16 or terminal 14 may generate an identifier for the answer, thus associating the identifier of the answer with the machine independent biometric behavior measurement data. The identifier for the answer is preferably made unique among all answers given in any answering session in which the physical information carrier 16 of the same user is used. In one example, the identifier for the answer comprises an identifier of the user, or the physical information carrier 16 and a time stamp of the answer.

Preferably, in sixth step 26 the identity of the user is also determined. This may be done by inputting a password (e.g. pin code) and verifying that this password is a password that has been defined for the user. Preferably, a biometric identification is performed to verify that the person that presents physical information carrier 16 is the user for which the biometric behavior template has been generated. Sixth step 26 may comprise a sub-step wherein terminal 14 or physical information carrier 16 verifies an identity of the user, for example by using biometric identification data measured by terminal 14 in sixth step 26 and comparison of the measured biometric identification data with the biometric identification data stored in physical information carrier 16.

In a seventh step 27, terminal 14 and/or physical information carrier 16 computes a likelihood or similar score value and/or determines a classification from the set of values of machine independent measurements of biometric behavior properties, under control of the parameters defined by the biometrics behavioral template stored in association with the verified user.

In an embodiment terminal 14 transmits the set of values for a question to physical information carrier 16 and the processor of physical information carrier 16 computes the likelihood or similar score value and/or determines a classification of the set of values using values of the parameters of the machine independent biometrics behavioral template from the memory of physical information carrier 16, without transmitting these values to terminal 14. In this way it is ensured that the value of the parameters of the machine independent biometrics behavioral template will not become known to terminal 14.

Alternatively, this may be ensured by performing different steps of the computation in terminal 14 and physical information carrier 16 respectively, wherein steps that involve access to information that discloses the value of the parameters or the classifications for which these parameters are used are performed by the processor of physical information carrier 16. Alternatively, this may be ensured by transmitting encrypted values of the parameters from physical information carrier 16 to terminal 14, and decrypting these values and performing computations of the likelihood or similar score value and/or classification using these values in a secured processor of terminal 14, or using a computation using the encrypted values (e.g. by means of a secure comparison protocol, and/or homomorphic encryption etc.). In the later alternative, the secured processor may alternatively be remote from terminal 14.

In an eight step 28, terminal 14 and/or physical information carrier 16 returns the likelihood or similar score value and/or a classification to the terminal 14. In a ninth step 29 terminal 14 executes an action based on the statement input in sixth step 26 and the likelihood or similar score value and/or a classification received in eight step 28. Alternatively, or in addition terminal 14 may be configured to generate an electronic certificate, linked to the statement wherein the likelihood or similar score value and/or a classification is recorded. The statement my be an electronic audio or video recording of speech of a person for example. The terminal may be configured to use a one way function or a cryptographic key based linking method to make subsequent tampering with the statement and/or the electronic certificate detectable. Methods of linking an electronic certificate to data in such a way are known per se.

When an experienced interrogator uses terminal 14 to question the user, it may be preferable to have a possibility to obtain likelihood or similar score value and/or classification for a plurality of different classes that relate to different aspects of the mental state of the user, which may be used as factors to determine whether the user believes that his or her answer is true. For example the classes may comprise a class of biometric behavior measurement values that are known to occur when the user hesitates, a class of biometric behavior measurement values that are known to occur when the user is tired, a class of biometric behavior measurement values that are known to occur when the user is angry, a class of biometric behavior measurement values that are known to occur when the user is solving a problem etc. The experienced interrogator may use information about such classifications to form his or her own opinion about lying by users with which the interrogator is not familiar. Optionally, the biometric behavior template may be dependent on the result of one or more of these classifications, and these results may also be used as input for the biometric behavior classification to determiner whether or not the measurements of the biometric behavior properties is in a class corresponding to the mental state of lying by the person.

In an alternative embodiment, terminal may be configured to select a suggestions from a predetermined set of interrogation strategies or from a predetermined set of types of questions based on such classification into such different classes, and output the selected suggestion to an inexperienced interrogator. This may be used to assist in bringing the interrogated person into a mental state wherein a subsequent state of believing or not believing the statement can be more reliably detected.

In an embodiment, terminal 14 and/or physical information carrier 16 may be configured to compute likelihood or similar score value and/or determine a classification for a plurality of such different classes, using values of the parameters of the machine independent biometrics behavioral template from the memory of physical information carrier 16.

In an embodiment, enrollment device 12 may be configured to operate as terminal 14 as well and vice versa at least some of terminals 14 may be configured to operate as enrollment device 12 as well. However, the same biometrics behavioral template in physical information carrier 16 may be used in all terminals. Terminals 14 are configured to operate independent on whether the biometrics behavioral template was obtained using measurements at the same terminal operating as enrollment device 12 or by another enrollment device 12.

In a further embodiment, it is made possible to use later obtained information whether the user lied or not when giving an answer to update the biometric behavior template. To do so, the set of values of machine independent biometric behavior measurement data associated with the answer is stored in association with the identifier of the answer, e.g. physical information carrier 16 or in central server 10. Later, when information is received that the answer was a lie or not, that information is also stored in association with the identifier of the answer, e.g. in central server 10.

Subsequently, when physical information carrier 16 is presented to an enrollment device 12 or a terminal 14, the enrollment device 12 or terminal 14 receives the information that the answer was a lie or not and, if not stored in physical information carrier 16, the behavior measurement data associated with that answer. In a step similar to third step 23 of figure 2, the biometric behavior template in physical information carrier 16 is updated using the information that the answer was a lie or not and the set of values of behavior measurement data associated with that answer. Further information such as the values of the parameters of the biometric behavior data stored in physical information carrier 16 may be used as well. Preferably, physical information carrier 16 stores more information for this purpose in addition to the template. For example, physical information carrier 16 may store machine independent sets of values biometric behavior measurement data associated with all answers, or with a number of most recent answers for use in the update. As another example, physical information carrier 16 may store indications of weight values to be given to the existing biometric behavior template and the new information.

In an embodiment, terminals 14 that are configured to trigger such updates are configured to do so when a physical information carrier 16 is presented, by requesting central server 10 to send the information that one or more answers that were given in sessions using that physical information carrier 16 were a lie or not and, if not already stored on physical information carrier 16, the sets of values behavior measurement data associated with those answers. Subsequently, such a terminal 14 and/or physical information carrier 16 executes the update the biometric behavior template in physical information carrier 16 using the received information.

Figure 4 shows a flow-chart of operation of a terminal 14 according to this embodiment. In a first step 41, terminal 14 detects the presentation of and physical information carrier 16 preferably verifies the identity of the user, e.g. based on biometric identification data from physical information carrier 16. In a second step 42, terminal 14 sends a message to central server 10, requesting information that one or more answers that were given in sessions using that physical information carrier 16 were a lie or not and the set of values of behavior measurement data associated with those answers. In a third step 43, terminal 14 receives a response to the message. If the response provides such information, terminal 14 and/or physical information carrier 16 executes a fourth step 44, similar to third step 23 of figure 2, and updates the parameters of the biometric behavior template in physical information carrier 16.

The method of updating the parameters depends on the classification method that will be used. For example, if support vector machine classification is used updated support vectors may be determined. If a neural network is used, a known neural network training method may be used. In other embodiments clustering may be used. If physical information carrier 16 stores machine independent biometric behavior measurement data associated with all previous answers and indications whether the used lied or not in at least part of the answers, the update may involve a fresh determination of the parameters.

After the process of figure 4, the terminal may proceed with the process of figure 3, using the updated parameters of the biometric behavior template.

Storage of the machine independent biometric behavior measurement data associated with different answers and the parameters of the biometrics behavior template in physical information carrier 16 has the advantage that it is easy to ensure that no unauthorized access to this information is possible. In other embodiments part or all of this information may be stored elsewhere, e.g. in encrypted form. In that case, the information may be downloaded to terminal 14 and decrypted, used and disposed of under control of the user, e.g. in response to biometric identification of the user.

Preferably, it is prevented that biometric behavior measurement data on one hand and classifications that have been associated with such data for training, or in the biometric behavior template on the other hand are not stored in the same storage device, or at storage devices where they can be accessed both without an act from the person. If the biometric behavior measurement data of examples for a user is stored in a storage device of server 10, the classifications associated with these examples is preferably stored elsewhere, where they cannot be accessed without an act from the person, e.g. in physical information carrier 16. Similarly, if the biometric behavior template provides for classes corresponding to lying and not lying, all or part of the parameters of the template may be stored in a storage device of server 10, and the classes associated with these examples are preferably identified elsewhere, where they cannot be accessed without an act from the person, e.g. in physical information carrier 16.

In other embodiments terminal 14 may send the machine independent biometric behavior measurement data associated with an answer to central server 10, and the server may be configured to perform seventh step 27 of the process figure 3 computes a likelihood or similar score value and/or determines a classification from the machine independent measurements, under control of the parameters defined by the biometrics behavioral template stored in association with the verified user. In this case, the server does not need information about the content of the answer, or the question to which it was an answer. The server merely computes a likelihood or similar score value and/or determines a classification and returns the result to the terminal. However, use of a server can make it more difficult to ensure protection against unauthorized use of the data.

Although embodiments have been described wherein terminals 14 have identical capabilities to measure values of biometric behavior properties, this is not indispensable. In an embodiment one or more advanced terminals may have one or more further sensors that are not present in the other terminals. Such advanced terminals may be configured to use initially a biometric behavior template prepared for the other terminals, recording, but ignoring, the measurements of the one or more further sensors. Subsequently, the process described with reference to figure 4 may be used to prepare a further biometric behavior template that involves measurements of the one or more further sensors and store this further biometric behavior template in the user domain storage device. The advanced terminals may be configured to use the further biometric behavior template when it is available.

More generally, in an embodiment, a plurality of biometric behavior templates for different combination of sensors may be stored in the user domain storage device. In this embodiment, at least one of the terminals may be configured to select one of the stored biometric behavior templates for use in the process to classify and/or compute a score function as described with reference to figure 3. Said at least one of the terminals may be configured to do so based on the combination of biometric behavior sensors that is available in the terminal, so that a stored biometric behavior template is selected that requires only measurements from biometric behavior sensors that are available in the terminal, and preferably all of these biometric behavior sensors, or at least as many as possible of these biometric behavior sensors. To establish a plurality of biometric behavior templates for this purpose, the template generation process as described with reference to figure 2, and/or the update process as described with reference to figure 4, may be executed a plurality of times, each for measurements from biometric behavior from a different set or sub-set of the biometric behavior sensors that is available in part or all of the training examples.

## Claims

1. A method of using a terminal to obtain authentication information associated with user input information, the method comprising
- measuring a set of values of biometric properties of behavior of a person at the terminal, using one or more biometric behavior sensors of the terminal;
- receiving an indication of a time interval from which the set of values has to be used for obtaining the authentication information for the user input information;
- reading parameters of a biometric of behavior template from a user domain storage device, to which access is enabled only under control of the person,
- wherein the parameters define a predetermined class of sets of values of biometric properties that have been determined to occur for the person when the person is in a predetermined type of mental state, or a computation for computing a score value from the set of values that the set of measured values belongs to said class;
- using the parameters in the terminal to determine whether or not the set of values in said time interval are within the predetermined class, and/or to compute a score value that the set of values in the time interval belongs to said class, or causing a computing device associated with the user domain storage device to do so.

2. A method according to claim 1, wherein the predetermined type of mental state is a state wherein the person believes that the input information is not true.

3. A method of using a system of terminals to obtain authentication information for user input information, wherein a first terminal of the system performs the method according to claim 1 or 2, the first terminal of the system further recording the set of values in association with an identifier of an event wherein the user input information was input, the method further comprising
- storing a subsequent feedback in association with the identifier in a server of the system, the subsequent feedback indicating that the person lied at the event;
- executing the method of claim 1 by a second terminal of the system, wherein, when the person gives the second terminal access to the user domain storage device, and before the second terminal uses the parameters are used in said step of using the parameters, the second terminal updates the parameters based on the recorded set of values in and the subsequent feedback associated with the identifier that was stored in the server, or causes the user domain storage device to do so.

4. A method according to claim 3, wherein the first terminal comprises an additional biometric behavior sensor of a type from which measurements are not used in the biometric behavior template, when the first terminal performs the method according to claim 1, the first terminal recording a further value, of a measurement determined using the additional biometric behavior sensor, in association with the identifier, said updating comprising determining parameters of a new biometric behavior template that involves measurements of the additional biometric behavior sensor.

5. A method according to any of the preceding claims, comprising biometric identification of the person before said using the parameters.

6. A method according to claim 5, wherein the user domain storage device stores biometric identification data of the person in association with the parameters of the biometric of behavior template, said biometric identification comprising measuring biometric identification data of the person when access is to the user domain storage device is provided, and comparing the measured biometric identification data with the biometric identification data of the person before using the associated parameters of the biometric of behavior template.

7. A method according to any of the preceding claims, wherein the user domain storage device is a portable physical information carrier supplied by the person, or a remote storage device to which access has been given under control of the person.

8. A method according to any of the preceding claims, comprising measuring biometric identification data of the person and comparing the measured biometric identification data with biometric identification data stored in the user domain storage device, the access to the user domain storage device being enabled dependent on whether the biometric identification data matches the stored biometric identification data.

9. A method according to any of the preceding claims, wherein the terminal records the user input information in association with an indication whether or not the set of values in said time interval is within the predetermined class, or computing a score value that the set of values in the time interval belongs to said class.

10. A terminal for obtaining authentication information associated with user input information, the terminal method comprising one or more biometric behavior sensors, a control interface, a processor and a communication unit for communicating with a user domain storage device, to which access is enabled only under control of a person, the processor being configured to
- obtain a set of values of biometric properties of behavior of the person at the terminal, using an output of the one or more biometric behavior sensors;
- receiving, from the control interface, an indication of a time interval from which the set of values has to be used for obtaining the authentication information for the user input information;
- using the communication unit to cause parameters of a biometric of behavior template to be read from the user domain storage device;
- wherein the parameters define a predetermined class of sets of values of biometric properties that have been determined to occur for the person when the person is in a predetermined type of mental state, or a computation for computing a score value from the set of values that the set of measured values belongs to said class;
- the terminal using the parameters to determine whether or not the set of values in said time interval are within the predetermined class, and/or to compute a score value that the set of values in the time interval belongs to said class, or causing a computing device associated with the user domain storage device to do so.

11. A system of terminals comprising a first and second terminal according to claim 10, method of using a system of terminals to certify user input information, wherein the first terminal is configured to record the set of values in association with an identifier of an event wherein the user input information was input, the system comprising a server configured for storing a subsequent feedback in association with the identifier, the subsequent feedback indicating that the person lied at the event;
- the second terminal being configured to updates the parameters based on the recorded set of values in and the subsequent feedback associated with the identifier that was stored in the server, or to cause the computing device associated with the user domain storage device to do so, when the person gives the second terminal access to the user domain storage device, and before the second terminal uses the parameters in said step of using the parameters.

12. A system according to claim 11, wherein the first terminal comprises an additional biometric behavior sensor of a type from which measurements are not used in the biometric behavior template, the first terminal being configured to record a further value, of a measurement determined using the additional biometric behavior sensor, in association with the identifier, said updating comprising determining parameters of a new biometric behavior template that involves measurements of the additional biometric behavior sensor.

13. A terminal according to claim 10, configured to measuring biometric identification data of the person to perform a biometric identification of the person, or causing the computing device associated with the biometric storage device to do so, by reading biometric identification data of the person from the user domain storage device and comparing the measured biometric identification data with the biometric identification data of the person before using the associated parameters of the biometric of behavior template.

14. A method according to any of the preceding claims, wherein the user domain storage device is a portable physical information carrier supplied by the person, or a remote storage device to which access has been given under control of the person.
